(19) Europäisches Patentamt
European Patent Office
Office européen des brevets

(11) Veröffentlichungsnummer: **0 203 607**
**B1**

(12) # EUROPÄISCHE PATENTSCHRIFT

(45) Veröffentlichungstag der Patentschrift: **31.08.88**

(21) Anmeldenummer: **86107327.8**

(22) Anmeldetag: **30.05.86**

(51) Int. Cl.⁴: **C 07 C 69/76**, C 07 C 67/00, A 01 N 37/10

(54) **Benzoesäureester, Verfahren zu ihrer Herstellung und ihre Verwendung zur Schädlingsbekämpfung.**

(30) Priorität: **30.05.85 DE 3519283**
**14.08.85 DE 3529126**

(43) Veröffentlichungstag der Anmeldung:
**03.12.86 Patentblatt 86/49**

(45) Bekanntmachung des Hinweises auf die Patenterteilung:
**31.08.88 Patentblatt 88/35**

(84) Benannte Vertragsstaaten:
**AT BE CH DE FR GB LI NL**

(56) Entgegenhaltungen:
**EP-A-0 156 263**
**DE-A-2 512 641**
**US-A-3 288 834**
**US-A-3 718 686**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch- Strasse 38, D-6700 Ludwigshafen (DE)**

(72) Erfinder: **Buerstinghaus, Rainer, Dr., Roentgenstrasse 38, D-6900 Heidelberg (DE)**
Erfinder: **Hofmeister, Peter, Dr., Bernard- Humblot-Strasse 12, D-6730 Neustadt (DE)**
Erfinder: **Seppelt, Wolfgang, Dr., Lucas- Cranach-Strasse 8, D-6712 Bobenheim- Roxheim (DE)**
Erfinder: **Hamprecht, Gerhard, Dr., Rote- Turm-Strasse 28, D-6940 Weinheim (DE)**
Erfinder: **Liese, Thomas, Dr., H 1,16/17, D-6800 Mannheim 1 (DE)**
Erfinder: **Adolphi, Heinrich, Dr., Kalmitweg 11, D-6703 Limburgerhof (DE)**
Erfinder: **Kuenast, Christoph, Dr., Muehlstrasse 21, D-6701 Waldsee (DE)**
Erfinder: **Neubauer, Hans- Jürgen, B 4,2, D-6800 Mannheim 1 (DE)**

**EP 0 203 607 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Es ist bekannt, daß Alkylester von Alkinsäuren oder Alkinylester geeignet sind zur Bekämpfung von Milben (US-A-4 024 278 bzw. US-A-3 996 380). Die Wirkung ist jedoch auf Arachniden beschränkt. Außerdem ist aus der DE-A-2 512 641 bekannt, daß 2,6-Dichlorbenzoesäurepropargylester als Pflanzenwachstumsregulator geeignet ist.

Es wurde gefunden, daß Benzoesäureester der Formel I

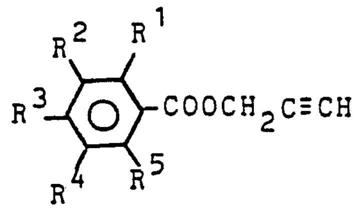

(I),

in der $R^1$, $R^2$, $R^3$, $R^4$, $R^5$ gleich oder verschieden sind und unabhängig voneinander für H, F, Cl, Br, J, verzweigtes oder unverzweigtes $C_nH_{2n+1}$ (mit n = 1 - 6), $OC_mH_{2m+1}$ (mit m = 1 - 4), -O-CO-$CH_3$, -$NO_2$, -CN, -$SCH_3$, -$SCCl_3$, -$SCF_3$, -$SCF_2Cl$, $CF_3$, -$CF_2H$, -$CCl_3$, -$CF_2Cl$, -$OCCl_3$, -$OCF_2Cl$, -$OCF_3$, -$OCF_2H$, -$OCF_2CF_2H$, -$OCF_2CFHCl$, -$OCF_2CFHBr$ stehen, $R^3$ außerdem für den Rest

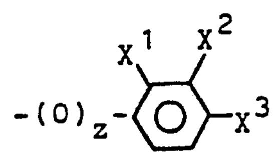

steht, in dem z die Zahl 1 oder 0 bedeutet und $X^1$, $X^2$, $X^3$, gleich oder verschieden sind und unabhängig voneinander, falls z = 1, für H, F, Cl, Br, -$NO_2$, $CH_3$, $C_2H_5$, -$OCH_3$, -$OC_2H_5$, -$CF_3$, -$OCF_3$, -$CF_2H$, -$CCF_2H$, -$CF_2Cl$, -$OCF_2Cl$, -$OCF_2CF_2H$, -OCF$_2$CFHCL, -$SCH_3$, $SCF_3$, stehen und $X^2$ zusammen mit $X^3$ für -$OCH_2O$- stehen kann, und falls z = 0 ist, $X^1$, $X^2$, $X^3$ gleich oder verschieden sind und unabhängig voneinander für H, F, Cl, $CF_3$, $CH_3$, $C_2H_5$, $OCH_3$ stehen

oder $R^2$ und $R^3$ gemeinsam die Molekülteile -O-$CF_2$-O- und -O-$CF_2$-$CF_2$-O- bedeuten,

mit der Maßgabe, daß $R^3$ nicht für F, -$OCH_3$ -$OC_2H_5$, -$OCF_3$, -O-$CH_2CF_2H$ stent wenn $R^1$, $R^2$, $R^4$ und $R^5$ jeweils Wasserstoff bedeuten und weiter mit der Maßgabe, daß $R^1$ und $R^5$ nicht gleichzeitig fur Chlor stehen, wenn $R^2$, $R^3$ und $R^4$ Waserstoff bedeuten, insektizid, akarizid und besonders ovizid bzw. ovolarvizid sehr gut wirksam und bekannten Wirkstoffen ähnlicher struktur bzw. gleicher Wirkungsrichtung überlegen sind.

Die Ester der Formel I können durch Umsetzung entsprechender Benzoesäuren II mit Propargylalkohol erhalten werden (vgl. Houben-Weyl, Methoden der organischen Chemie, Band VIII, S. 516 ff., Georg-Thieme-Verlag, Stuttgart 1952).

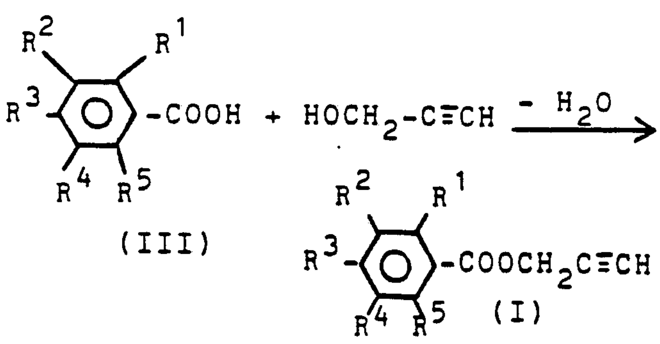


(III)

(I)

Die Umsetzung kann in an sich bekannter Weise durch Zusatz von Katalysatoren wie Schwefelsäure, Halogenwasserstoff, Sulfonsäuren, sauren Ionenaustauschern beschleunigt und das Veresterungsgleichgewicht in gewünschtem Sinne verschoben werden, indem man dem Reaktionsgemisch das Wasser oder den Ester I entzieht, z. B. durch azeotrope Destillation oder durch Bindung des Wassers an Schwefel- oder Halogenwasserstoffsäure.

Ebensogut kann man die entsprechenden Säurehalogenide III, in denen Hal(ogen) für Fluor, Chlor und Brom steht, mit Propargylalkohol in Gegenwart eines Säureacceptors umsetzen (vgl. Houben Weyl, Methoden der organischen Chemie, Band VIII, S. 543 gg., Georg-Thieme-Verlag, Stuttgart 1952).

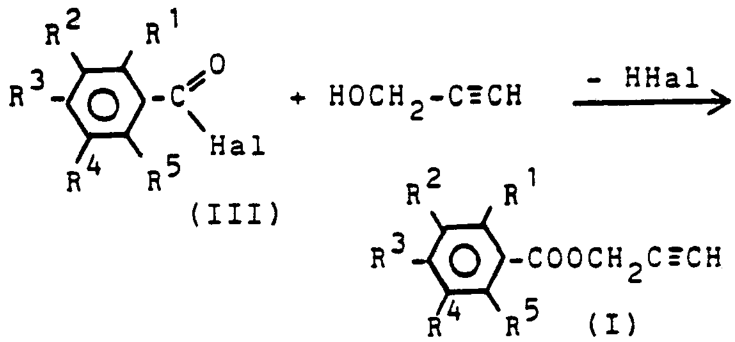


(III)

(I)

Als Säurebindemittel eignen sich die üblichen basischen Mittel, insbesondere aliphatische, aromatische und heterocyclische Amine, z. B. Triethylamin, Dimethylamin, Piperidin, Diazabicyclooctan, Dimethylanilin, Dimethylbenzylamin, Pyridin, Lutidin, Dimethylaminopyridin. Auch Alkalimetallcarbonate, wie Natrium- oder Kaliumcarbonat eignen sich als Säureacceptoren.

Die Umsetzung kann in einem Lösungs- oder Verdünnungsmittel ausgeführt werden. Hierzu sind teilweise die angeführten Säureacceptoren selbst oder beispielsweise folgende Lösungs- oder Verdünnungsmittel oder Gemische derselben geeignet:

Aliphatische und aromatische, gegebenenfalls chlorierte Kohlenwasserstoffe, wie Petrolether, Benzol, Toluol, Xylol, Benzin, Dichlormethan, Chloroform, Tetrachlormethan, 1,2-Dichlormethan, Chlorbenzol, Ether wie Di-ethyl- und Di-n-butylether, Methyl-tert.-butylether, Tetrahydrofuran, Dioxan; Ketone beispielsweise Aceton, Methylethylketon, Methylisopropylketon; ferner Nitrile wie Acetonitril und Propionitril.

Üblicherweise setzt man die Ausgangsstoffe in stöchiometrischem Verhältnis ein. Ein Überschuß des einen oder anderen kann in Einzelfällen aber durchaus vorteilhaft sein.

Die Umsetzung verläuft gewöhnlich oberhalb von 0° C mit ausreichender Geschwindigkeit. Da sie meist unter Wärmeentwicklung verläuft, kann es von Vorteil sein, eine Kühlmöglichkeit vorzusehen.

Die erfindungsgemäßen Benzoesäureester können außerdem noch nach praktisch allen bekannten Verfahren der Estersynthese hergestellt werden, beispielsweise durch Umsetzung von entsprechenden Benzoesäureanhydriden mit Propargylalkohol, durch Umsetzung von entsprechenden Benzoesäuresalzen mit Propargylhalogeniden oder durch Umesterungsreaktionen (vgl. Houben-Weyl a.a. O., S. 508 - 628; C. Ferri, Reaktionen der organischen Synthese, S. 446 ff., Georg-Thieme-Verlag, Stuttgart 1978; S. Patai, The Chemistry of Carboxylic acids and Esters, S. 505 ff., Interscience Publishers, London 1969).

Die als Ausgangsmaterial benötigten Benzoesäuren der Formel II sind größtenteils bekannt oder handelsüblich; andernfalls lassen sie sich in an sich bekannter Weise nach den zur Synthese substituierter Benzoesäuren geeigneten Verfahren aus entsprechenden Vorstufen (beispielsweise Benzoesäureestern, Benzonitrile, Benzaldehyde, Toluole, Trifluormethylbenzole, Halogenbenzole, Benzylalkohole, Benzylhalogenide, Arylmethylketone) herstellen (vgl. Tietze/Eicher, Reaktionen und Synthesen, S. 102 ff., Georg-Thieme-Verlag, Stuttgart 1981; M. Hudlicky, Chemistry of Organic Fluorine Compounds, S. 704, John Wiley & Sons, New York 1976); die aus ihnen in einigen Fällen hergestellten Säurehalogenide wurden z. T. nach Houben-Weyl, a.a. O., S. 463 - 476 erhalten.

Die Verbindungen der Formel I fallen meist als ölige Substanzen oder als Feststoffe an. Im folgenden werden daher von den jeweils hergestellten Stoffen die Schmelzintervalle, die Brechungsindizes oder IR-Spektren mit typischen Absorptionsmaxima aus dem sogenannten "fingerprint"-Bereich zwischen 1400 $cm^{1}$ und 800 cm-1 angegeben;

**Beispiel 1**

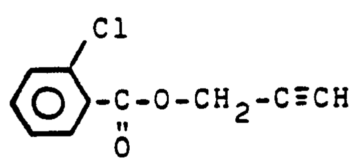

Eine Lösung von 15,65 g 2-Chlorbenzoesäure, 16,8 g Propargylalkohol und 0,6 g p-Toluolsulfonsäuremonohydrat in 200 ml Chloroform wird am Wasserabscheider 9 Stunden unter Rückfluß erhitzt. Die Reaktionslösung wird nach dem Erkalten mit je 100 ml Wasser, 10prozentiger $NaHCO_3$-Lösung und nochmals Wasser gewaschen und das Solvens am Rotationsverdampfer abgezogen. Nach Umkristallisation des Rückstandes aus Methanol/Wasser (2 : 1) erhält man 14,2 g 2-Chlorbenzoesäurepropargylester als farblose

Kristalle, die zwischen 59 und 60°C schmelzen. Ausbeute 73 % der rechnerisch möglichen.

Infrarotabsorptionen ($cm^{-1}$): 1293, 1271, 1258, 1138, 1123, 1052, 748.

**Beispiel 2**

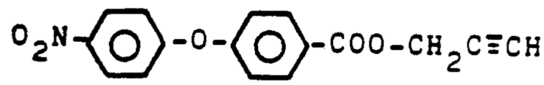

4,20 g Propargylalkohol und 3,95 g Pyridin werden in 100 ml trockenem Tetrahydrofuran gelöst. Unter Eiskühlung fügt man tropfenweise eine Lösung von 13,85 g 4-(4-Nitrophenoxy)benzoylchlorid in 30 ml Tetrahydrofuran hinzu. Man läßt die Mischung 8 Stunden bei 20°C rühren, filtriert das Pyridinhydrochlorid ab, engt das Filtrat im Vakuum ein und behandelt den Rückstand mit 150 ml Essigester. Die organische Phase wird viermal mit Wasser gewaschen, über Natriumsulfat getrocknet und vom Solvens befreit. Es verbleiben 13,5 g 4-(4-Nitrophenoxy)benzoesäurepropargylester als gelbes Pulver. Fp.: 88°C. Ausbeute: 91 % der theoretisch möglichen.

Infrarotabsorptionen ($cm^{-1}$): 1503, 1489, 1345, 1280, 1249, 1096.

**Beispiel 3**

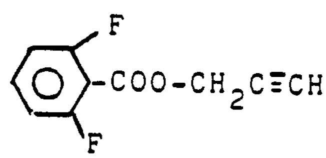

5,0 g Propargylalkohol werden in 50 ml Pyridin gelöst. Nach dem Zutropfen von 14,1 g 2,6-Difluorbenzoylchlorid bei maximal 0°C läßt man die Mischung noch 12 Stunden bei Raumtemperatur rühren. Man fügt 50 ml Diethylether hinzu, saugt das Pyridinhydrochlorid ab und engt das Filtrat im Wasserstrahlvakuum ein. Der Rückstand wird in 150 ml Essigester aufgenommen, dreimal mit Wasser geschüttelt und die organische Phase über Natriumsulfat getrocknet. Danach werden die leicht flüchtigen Bestandteile, zuletzt bei 40°C und 1 mbar abgezogen. Man erhält 14,1 g (90 % der stöchiometrischen Menge) 2,6-Difluorbenzoesäurepropargylester als nahezu farbloses Öl mit einem Brechungsindex von $n^{23}_D = 1{,}4960$.

**Beispile 4**

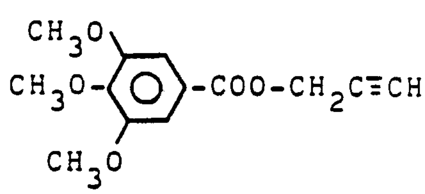

Die Lösung von 3,2 g Propargylalkohol in 35 ml absolutem Pyridin wird auf 5°C gekühlt und bei dieser Temperatur portionsweise mit 12 g 3,4,5-Trimethoxybenzoylchlorid versetzt. Man läßt noch 8 Stunden bei Raumtemperatur rühren, gießt das Reaktionsgemisch in 100 ml Eiswasser und stellt mit conc. Salzsäure auf pH 2 ein. Die wäßrige Phase wird dreimal mit Ether extrahiert und die vereinigten Etherextrakte mit 10prozentiger $NaHCO_3$-Lösung und Wasser gewaschen. Nach Trocknen über Natriumsulfat und Abziehen des Solvens erhält man 11,5 g 3,4,5-Trimethoxybenzoesäurepropargylester. Fp.: 78 - 79°C. Ausbeute: 88 % der rechnerisch möglichen.

Die in der nachstehenden Tabelle aufgeführten Verbindungen wurden ebenfalls auf den in den Beispielen 1 - 4 beschriebenen Wegen erhalten, soweit mindestens eine physikalische Angabe zu ihrer Identifizierung

vorliegt; andere Verbindungen, die der Formel (I) entsprechen und z. B. in dieser Tabelle ohne nähere Angaben aufgeführt sind, können auf die gleiche Weise unter entsprechender Abwandlung der Vorschriften nach der jeweils benötigten Menge und (wegen der besten Reaktionsbedingungen) gegebenenfalls nach einem Vorversuch erhalten werden.

**Tabelle**

| Bsp. Nr. | $R^1$ | $R^2$ | $R^3$ | $R^4$ | $R^5$ | physikalische Angaben |
|---|---|---|---|---|---|---|
| 5 | H | $CF_3$ | H | H | H | $n^{23}_D$: 1,4681 |
| 6 | H | Cl | Cl | H | H | Fp.: 35 - 36° C |
| 7 | Cl | H | Cl | H | H | Fp.: 27 - 28° C |
| 8 | H | H | Br | H | H | Fp.: 30 - 32° C |
| 9 | H | H | H | H | Br | 1433, 1287, 1249, 1107, 744 $cm^{-1}$ |
| 10 | H | $OCH_3$ | H | $CCH_3$ | H | Fp.: 72° C |
| 11 | H | F | H | H | H | 1291, 1271, 1095, 754 $cm^{-1}$ |
| 12 | H | $CCl_3$ | H | H | H | 1271, 1173, 733 $cm^{-1}$ |
| 13 | H | H | Cl | H | H | 1596, 1269, 1107, 758 $cm^{-1}$ |
| 14 | H | Cl | H | Cl | H | 1262, 1139, 806, 762 $cm^{-1}$ |
| 16 | H | Cl | H | H | H | Fp.: 31 - 33° C |
| 17 | $OCOCH_3$ | H | H | H | H | Fp.: 71° C |
| 18 | H | H | $NO_2$ | H | Cl | Fp.: 69° C |
| 19 | $CF_3$ | H | H | H | H | 1312, 1289, 1261, 1167, 1144, 1104, 1053, 1037, 720 $cm^{-1}$ |
| 20 | $NO_2$ | $CH_3$ | H | H | H | Fp.: 81° C |
| 21 | H | $OCH_3$ | $OCH_3$ | H | $NO_2$ | Fp.: 90 - 92° C |
| 22 | H | H | $NO_2$ | O-n-$C_4H_9$ | H | Fp.: 72° C |
| 23 | $NO_2$ | H | H | H | H | Fp.: 56 - 57° C |
| 24 | H | H | H | $NO_2$ | H | Fp.: 80 - 81° C |
| 25 | H | $NO_2$ | H | $NO_2$ | H | Fp.: 88 - 89° C |
| 26 | H | H | $NO_2$ | H | H | Fp.: 94 - 95° C |
| 27 | H | H | 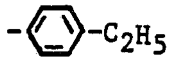 | H | H | |
| 28 | H | H | $CCl_3$ | H | H | $n^{23}_D$: 1,5589 |
| 29 | H | H | $OCH_3$ | $OCH_3$ | H | Fp.: 65 - 69° C |
| 30 | H | H | $OCF_2H$ | H | H | $n^{25}_D$: 1,4960 |
| 31 | H | H | $SCF_3$ | H | H | $n^{25}_D$: 1,5020 |
| 32 | H | H | F | F | H | $n^{25}_D$: 1,4950 |
| 33 | H | $OCF_3$ | H | H | H | $n^{25}_D$: 1,4618 |
| 34 | H | Cl | O-$CCl_3$ | H | H | $n^{25}_D$: 1,5550 |
| 35 | H | H | O-$CF_2Cl$ | H | H | $n^{25}_D$: 1 4983 |
| 36 | J | H | H | H | H | Fp.: 45° C |
| 37 | H | H | H | $CF_3$ | H | $n^{26}_D$: 1,4705 |
| 38 | H | H | H | H | F | $n^{25}_D$: 1,5145 |
| 39 | H | H | -$C(CH_3)_3$ | H | H | $n^{25}_D$: 1,5161 |
| 40 | $CH_3$ | H | $CH_3$ | H | $CH_3$ | $n227_D$: 1,5170 |
| 41 | $CH_3$ | H | H | H | H | Fp.: 35° C |
| 42 | H | H | n-$C_6H_{13}$ | H | H | $n^{26}_D$: 1,5091 |
| 43 | H | H | 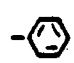 | H | H | Fp.: 107° C |
| 44 | H | H | H | H | H | $n^{27}_D$: 1,5288 |
| 45 | H | H | $CH_3$ | H | H | $n^{26}_D$: 1,5299 |
| 46 | H | $CH_3$ | H | H | H | $n^{27}_D$: 1,5270 |
| 47 | $CF_3$ | H | H | H | H | |
| 48 | $OCF_3$ | H | H | H | H | |
| 49 | H | H | $CF_3$ | H | H | |
| 50 | H | Cl | F | Cl | H | |

| | | | | | | |
|---|---|---|---|---|---|---|
| 51 | H | H | $CF_2H$ | H | H | |
| 52 | H | H | F | $NO_2$ | H | |
| 53 | H | F | $NO_2$ | H | H | |
| 54 | H | H | Cl | $CF_3$ | H | |
| 55 | H | Cl | $CF_3$ | H | H | |
| 56 | H | Cl | H | $CF_3$ | H | |
| 57 | H | $CF_3$ | Cl | Cl | H | |
| 58 | H | Cl | $CF_3$ | Cl | H | |
| 59 | H | H | $OCF_3$ | $NO_2$ | H | |
| 60 | H | Br | $OCF_3$ | H | H | |
| 61 | H | $NO_2$ | $OCF_3$ | Cl | H | |
| 62 | H | H | F | Br | H | |
| 63 | H | H | Br | F | H | |
| 64 | H | Cl | $OCF_3$ | H | H | |
| 65 | H | $CF_3$ | Cl | H | H | |
| 66 | H | Cl | $OCF_2Cl$ | H | H | |
| 67 | H | H | F | Cl | H | |
| 68 | F | F | F | F | F | |
| 69 | H | Cl | F | Br | H | |
| 70 | H | F | F | F | H | |
| 71 | H | $CF_3$ | $CF_3$ | H | H | |
| 72 | H | $CF_3$ | H | $CF_3$ | H | |
| 73 | H | Br | F | Br | H | |
| 74 | H | $-O-CF_2-O-$ | H | H | H | |
| 75 | H | H | Br | $OCF_3$ | H | |
| 76 | H | $SCF_3$ | H | H | H | |
| 77 | H | H | $CF_2Cl$ | H | H | |
| 78 | H | H | $CF_3$ | H | Cl | |
| 79 | H | $CHF_2$ | H | H | H | |
| 80 | H | H | F | H | F | $n^{25}_D$: 1,5051 |
| 81 | H | H | Cl | H | F | Fp.: 31-34° C |
| 82 | H | H | $NO_2$ | H | F | |
| 83 | H | F | $CH_3$ | H | H | $n^{21}_D$: 1,5164 |
| 84 | H | F | $OCH_3$ | H | H | |
| 85 | Br | H | F | H | H | |
| 86 | F | H | Br | H | H | |
| 87 | Cl | H | H | F | H | |
| 88 | Cl | H | H | H | F | |
| 89 | $OCF_2CF_2H$ | H | H | H | H | |
| 90 | H | H | F | H | Cl | $n^{20}_D$: 1,5276 |
| 91 | H | H | F | H | $NO_2$ | |
| 92 | F | H | $OCH_3$ | H | H | |
| 93 | H | Cl | $OCH_3$ | H | H | |
| 94 | Cl | H | $OCH_3$ | H | H | |
| 95 | H | H | 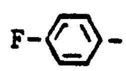 | H | H | |
| 96 | H | H | 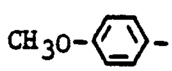 | H | H | |
| 97 | H | H | 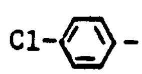 | H | H | |
| 98 | H | H | $S-CCl_3$ | H | H | $n^{25}_D$: 1,5868 |
| 99 | H | H | $S-CF_2Cl$ | H | H | |
| 100 | H | H | $CH_3S$ | H | H | Fp.: 48 - 50° C |
| 101 | H | H | J | H | H | |
| 102 | $CH_3$ | H | F | H | H | |
| 103 | H | H | $CH_3$ | H | $CH_3$ | |
| 104 | H | H | 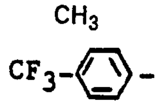 | H | H | |
| 105 | H | H | 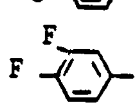 | H | H | |

| | | | | | | |
|---|---|---|---|---|---|---|
| 106 | H | H | 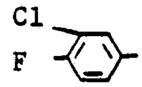 | H | H | |
| 107 | H | H | 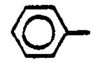 | F | H | Fp.: 39-40°C |
| 108 | H | H | 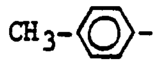 | H | H | |
| 109 | H | H | 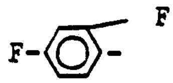 | H | H | |
| 110 | $CF_3$ | H | Cl | H | H | |
| 111 | Br | H | $CF_3$ | H | H | |
| 112 | $CF_3$ | H | Br | H | H | |
| 113 | H | H | 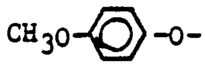 | H | H | Fp.: 41-42°C |
| 114 | H | H | 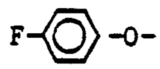 | H | H | Fp.: 59°C |
| 115 | H | H | 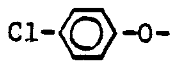 | H | H | Fp.: 73°C |
| 116 | H | H | 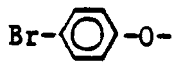 | H | H | Fp.: 75-76°C |
| 117 | H | H | 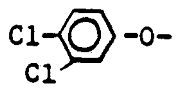 | H | H | |
| 118 | H | H | 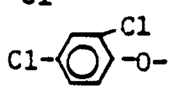 | H | H | |
| 119 | H | H | 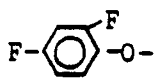 | H | H | |
| 120 | H | H | 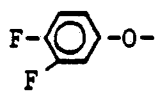 | H | H | |
| 121 | H | H | 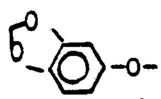 | H | H | 1228, 1174, 1164<br>1109, 1096 $cm^{-1}$ |
| 122 | H | H | 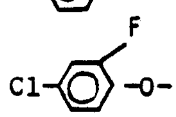 | H | H | |
| 123 | H | H | 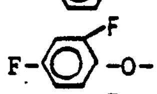 | H | H | |
| 124 | H | H | 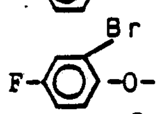 | H | H | |
| 125 | H | H | 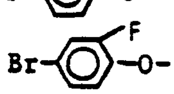 | H | H | |
| 126 | H | H | 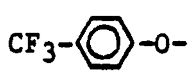 | H | H | |
| 127 | H | H | 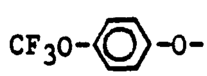 | H | H | Fp.: 56 - 57°C |
| 128 | H | H | 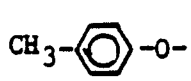 | H | H | Fp.: 47 - 48°C |
| 129 | H | H | 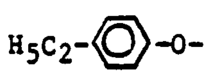 | H | H | |

| | | | | | | |
|---|---|---|---|---|---|---|
| 130 | H | H | 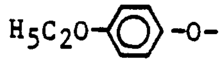 | H | H | |
| 131 | H | H | 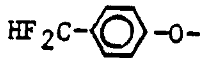 | H | H | |
| 132 | H | H | 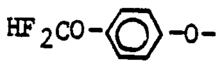 | H | H | |
| 133 | H | H | 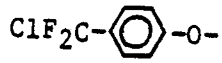 | H | H | |
| 134 | H | H | 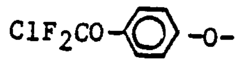 | H | H | |
| 135 | H | H | 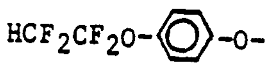 | H | H | |
| 136 | H | H | 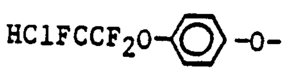 | H | H | |
| 137 | H | H | 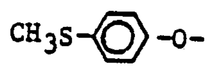 | H | H | Fp.: 47-48°C |
| 138 | H | H | 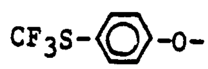 | H | H | |
| 139 | H | H | $-OCF_2CF_2H$ | H | H | |
| 140 | H | H | $O-CF_2CFClH$ | H | H | |
| 141 | H | H | $O-CF_2CFBrH$ | H | H | |
| 142 | H | H | $O-CF_2CF_2H$ | H | Cl | |
| 143 | $O-CF_2CFHCl$ | H | H | H | H | |
| 144 | $O-CF_2FHBr$ | H | H | H | H | |
| 145 | H | H | CN | H | H | |
| 146 | H | H | H | H | CN | |
| 147 | H | H | $OCCl_3$ | H | H | $n^{25}_D$: 1,5492 |
| 148 | H | Cl | 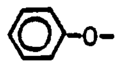 | H | H | |
| 149 | H | Cl | 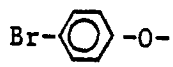 | H | H | $N^{21}_D$: 1,5968 |
| 150 | H | Cl | 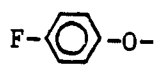 | H | H | Fp.: 79- 81°C |
| 151 | H | Cl | 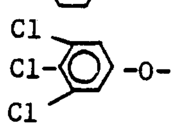 | H | H | Fp.: 99 - 104°C |
| 152 | H | Cl | 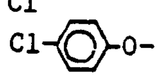 | H | H | Fp.: 50 - 53°C |
| 153 | H | Cl | 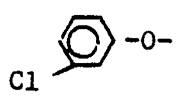 | H | H | |

Die vorstehend aufgeführten und andere erfindungsgemäße Wirkstoffe werden auf die für Insektizide übliche Weise angewendet. Angaben zur Formulierung, Anwendungstechnik und Wirkungsweise und Angaben über geeignete Mischungspartner zur Erzielung synergistischer und anderer vorteilhafter Wirkungen können beispielsweise der US-PS 4 320 122 entnommen werden.

In den folgenden Anwendungsbeispielen wurden als Vergleichsmittel eingesetzt

I 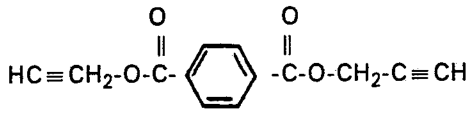

und

II 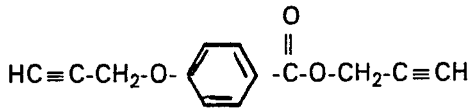

beide aus US 3 996 380

**Zuchtversuch mit Baumwollwanzen (Dysdercus intermedius)**

Baumwollwanzen Dysdercus intermedius werden im 4. Larvenstadium in Petrischalen (Ø 10 cm) dem Wirkstoffbelag der Testsubstanzen für 24 Stunden ausgesetzt.

Die Überlebenden züchtet man in 1 l-Gläsern auf feuchtem Quarzsand, der vorher mit der Wirkstofflösung versetzt wurde bis zum Schlüpfen der $F_1$-Generation.

Dabei entspricht eine Wirkstoffmenge von z. B. 2,5 mg/Schale einer Konzentration von 25 ppm (im Sand) usw.

Beurteilt wird Mortalität und Vermehrung. Bei diesem Test wirkt eine repräsentative Auswahl der erfindungsgemäßen Verbindungen in einer Konzentration von 10 bis 25 ppm total, während die Vergleichsmittel I und II bei einer Konzentration von 25 ppm weniger als 60 % Mortalität zur Folgen haben.

**Wirkung auf Eier der Baumwollwanze (Dysdercus intermedius)**

Ca. 200 frisch abgelegte Eier der Baumwollwanze heftet man auf Klebestreifen und taucht diese in die wäßrige Wirkstoffaufbereitung. Darauf lagert man die Streifen bei 25°C und 70 % Luftfeuchtigkeit bis zum Schlüpfen der unbehandelten Kontrolle.

Die erfindungsgemäßen Wirkstoffe haben i.a. bei Anwendung in 0,02 %-iger Lösung 100 %-ige Mortalität zur Folge, während Vergleichsmittel I in 0,04 %-iger Lösung ca. 80 % Mortalität erzielt.

**Plutella maculipennis, Kohlschaben-Raupen; Fraß- und Kontaktwirkung**

Blätter von jungen Kohlpflanzen werden 3 Sekunden in die wäßrige Wirkstoffemulsion getaucht und nach kurzem Abtropfen auf einen angefeuchteten Filter in eine Petrischale gelegt. Das Blatt wird darauf mit 10 Raupen des 4. Stadiums belegt.

Nach 48 Stunden beurteilt man die Wirkung.

Die erfindungsgemäßen Verbindungen wirken als 0,1 %-ige Lösung i.a. total; die Vergleichsmittel bei der gleichen Konzentration zu weniger als 60 %.

**Heliothis virescens; ovo-larvizide Wirkung**

Behandelte Blattstanzstücke (Ø 22 mm) belegt man mit 15 frischen abgelegten Eiern von Heliothis virescens.

Nach 4 Tagen beurteilt man Schlupf und Mortalität.

Die Wirkstoffe erreichen in 0,1 %-iger Lösung i.a. totalen Wirkungserfolg.

## Patentansprüche

1. Benzoesäureester der Formel I

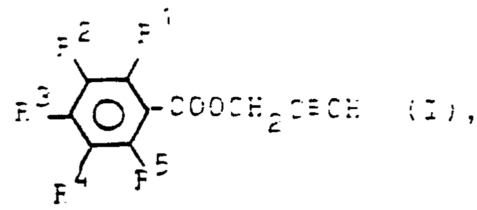

in der $R^1$, $R^2$, $R^3$, $R^4$, $R^5$ gleich oder verschieden sind und unabhängig voneinander für H, F, Cl, Br, J, verzweigtes oder unverzweigtes $C_nH_{2n+1}$ (mit n = 1 - 6), $OC_mH_{2m+1}$ (mit m =1 - 4), $-O-CO-CH_3$, $-NO_2$, -CN, $-SCH_3$, $-SCCl_3$ $-SCF_3$, $-SCF_2Cl$, $-CF_3$ $-CF_2H$, $-CCl_3$, $-CF_2Cl$, $-OCCl_3$ $-OCF_2Cl$, $-OCF_3$, $-OCF_2H$, $-OCF_2CF_2H$, $-CCF_2CFHCl$, $-OCF_2CFHBr$ stehen,

$R^3$ außerdem für den Rest

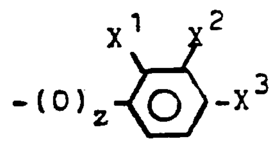

steht, in dem z die Zahl 1 oder 0 bedeutet und $X^1$, $X^2$, $X^3$ gleich oder verschieden sind und unabhängig voneinander, falls z = 1, für H, F, Cl, Br, $-NO_2$, $CH_3$, $C_2H_5$, $-OCH_3$, $-OC_2H_5$, $-CF_3$, $-OCF_3$, $-CF_2H$, $-OCF_2H$, $-CF_2Cl$, $-OCF_2Cl$, $-OCF_2CF_2H$, -OCF$_2$CFHCL, $-SCH_3$, $SCF_3$ stehen oder $X^2$ zusammen mit $X^3$ für $-OCH_2O-$ stehen und falls z = 0 ist, für H, F, Cl, $CF_3$, $CH_3$, $C_2H_5$, $OCH_3$ stehen,

oder $R^2$ und $R^3$ gemeinsam die Molekülteile $-O-CF_2-O-$ und $-O-CF_2-CF_2-O-$bedeutet, mit der Maßgabe, daß $R^3$ nicht für F, $-OCH_3$, $-OC_2H_5$, $-OCF_3$, $-O-CF_2CF_2H$ steht, Wenn $R^1$, $R^2$, $R^4$ und $R^5$ jeweils Wasserstoff bedeuten, und weiter mit der Maßgabe, daß $R^1$ und $R^5$ nicht gleichzeitig für Chlor stehen, wenn $R^2$, $R^3$ und $R^4$ Wasserstoff bedeuten.

2. Verfahren zur Herstellung der Benzoesäureester der Formel I gemäß Anspruch 1, dadurch gekennzeichnet, daß man entsprechende Benzoesäuren II

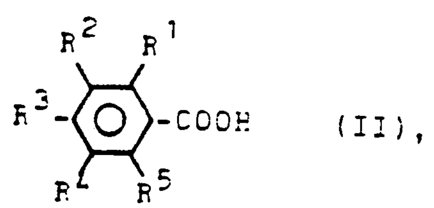

mit Propargylalkohol umsetzt oder daß man entsprechende Säurehalogenide III

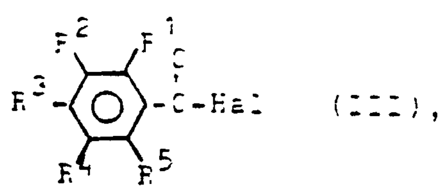

mit Propargylalkohol in Gegenwart eines Säureacceptors gegebenenfalls in Gegenwart eines Lösungs- oder Verdünnungsmittels umsetzt.

3. Schädlingsbekämpfungsmittel, enthaltend eine Verbindung gemäß Anspruch 1.

4. Schädlingsbekämpfungsmittel, enthaltend einen festen oder flüssigen Trägerstoff und mindestens einen Benzoesäureester der Formel I.

5. Verwendung von Benzoesäureestern der Formel I gemäß Anspruch 1 - 3 als Insektizid, Akarizid, insbesondere Ovizid.

6. Verfahren zur Bekämpfung von Schädlingen, dadurch gekennzeichnet, daß man eine wirksame Menge des Benzoesäureesters der Formel I gemäß Anspruch 1 - 3 auf Schädlinge bzw. deren Lebensraum einwirken läßt.

**Claims**

1. A benzoic ester of the formula I

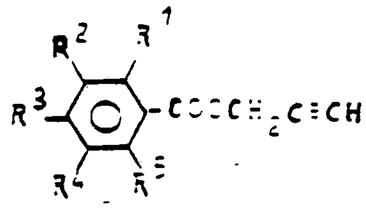

(I),

where $R^1$, $R^2$, $R^3$, $R^4$ and $R^5$ are identical or different and independently from one another are each H, F, Cl, Br, I, branched or straight-chain $C_nH_{2n+1}$ (where n is from 1 to 6), $OC_mH_{2m+1}$ (where m is from 1 to 4), $-O-CO-CH_3$, $-NO_2$, -CN $-SCH_3$ $-SCCl_3$, $-SCF_3$, $-SCF_2Cl$, $CF_3$, $-CF_2H$, $-CCl_3$, $-CF_2Cl$, $-OCCl_3$, $-OCF_2Cl$, $-OCF_3$, $-OCF_2H$, $-OCF_2CF_2H$, $-OCF_2CFHCl$ or $-OCF_2CFHBr$, $R^3$ may furthermore be a radical

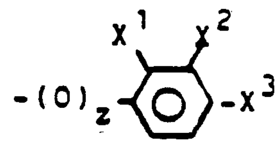

where z is 1 or 0 and $X^1$, $X^2$ and $X^3$ are identical or different and, if z is 1, independently of one another are each H, F, Cl, Br, $-NO_2$, $CH_3$, $C_2H_5$, $-OCH_3$, $-OC_2H_5$ $-CF_3$, $-OCF_3$, $-CF_2H$, $-OCF_2H$, $-CF_2Cl$, $-OCF_2Cl$, $-OCF_2CF_2H$ $-OCF_2CFHCl$, $-SCH_3$ or $SCF_3$, and $X^2$ together with $X^3$ may be $-OCH_2O-$, and if z is 0, $X^1$, $X^2$ and $X^3$ are identical or different and independently of one another are each H, F, Cl, $CF_3$, $CH_3$, $C_2H_5$ or $OCH_3$, or $R^2$ and $R^3$ together may form the molecular moiety $-O-CF_2-O-$ or $-O-CF_2-CF_2-O-$, with the proviso that $R^3$ is not F, $-OCH_3$, $OC_2H_5$, $-OCF_3$ or $-O-CF_2CF_2H$ when $R^1$, $R^2$, $R^4$ and $R^5$ are each hydrogen, and with the further proviso that $R^1$ and $R^5$ are not both simultaneously chlorine when $R^2$, $R^3$ and $R^4$ are each hydrogen.

2. A process for the preparation of a benzoic ester of the formula I as claimed in claim 1, wherein an appropriate benzoic acid II

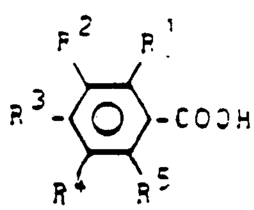

(II),

is reacted with propargyl alcohol, or an appropriate acyl halide III

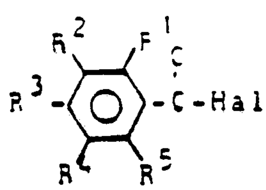

(III),

is reacted with propargyl alcohol in the presence of an acid acceptor and in the presence or absence of a solvent or diluent.

3. A pesticide containing a compound as claimed in claim 1.

4. A pesticide containing a solid or liquid carrier and at least one benzoic ester of the formula I.

5. Use of a benzoic ester of the formula I as claimed in any of claims 1 to 3 as an insecticide, acaricide and particularly ovicide.

6. A process for combating pests, wherein an effective amount of a benzoic ester of the formula I as claimed in any of claims 1 to 3 is allowed to act on pests or their habitat.

**Revendications**

1. Ester benzoïque de formule I

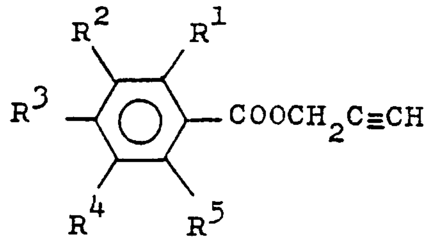

(1),

dans laquelle $R^1$, $R^2$, $R^3$, $R^4$, $R^5$ sont identiques ou différents, et sont mis, indépendamment l'un de l'autre, pour H, F, Cl, Br, I, $C_nH_{2+1}$ (avec n = 1 - 6) ramifié ou non ramifié, $OC_mH_{2+1}$ (avec m = 1 - 4), $-O-CO-CH_3$, $-NO_2$, -CN, $-SCH_3$, $-SCCl_3$, $-SCF_3$, $-SCF_2Cl$, $CF_3$, $-CF_2H$, $-CCl_3$, $-CF_2Cl$, $-OCCl_3$, $-OCF_2Cl$, $-OCF_3$, $-OCF_2H$, $-OCF_2CF_2H$, $-OCF_2CFHCl$, $-OCF_2CFHBr$,

$R^3$, en outre, est mis pour le reste

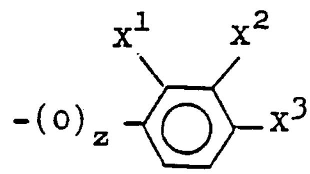

dans lequel z est le nombre 1 ou 0 et $X^1$, $X^2$, $X^3$ sont identiques ou différents et représentent, indépendamment l'un de l'autre, lorsque z = 1, H, F, Cl, Br, $-NO_2$, $CH_3$, $C_2H_5$, $-OCH_3$, $-OC_2H_5$, $-CF_3$, $-OCF_3$, $-CF_2H$, $-OCF_2H$, $-CF_2Cl$, $-OCF_2Cl$, $-OCF_2CF_2H$, $-OCF_2CFHCl$, $-SCH_3$, $SCF_3$ où $X^2$ avec $X^3$ représentent $-OCH_2O-$ et, lorsque z = 0, représentent H, F, Cl, $CF_3$, $CH_3$, $C_2H_5$, $OCH_3$,

où $R^2$ et $R^3$, ensemble, signifient la pertie de molécule $-O-CF_2-O-$ et $-O-CF_2-CF_2-O$, sous réserve que $R^3$ ne représente pas F, $-OCH_3$, $-OC_2H_5$, $-OCF_3$, $-O-CF_2CF_2H$, lorsque $R^1$, $R^2$, $R^4$ et $R^5$ signifient respectivement hydrogène, et, en outre, sous réserve que $R^1$ et $R^5$ ne représentent pas simultanément chlore, quand $R^2$, $R^3$ et $R^4$ représentent hydrogène.

2. Procédé de préparation d'ester benzoïque de formule I selon la revendication 1, caractérisé par le fait que l'on fait réagir de l'acide benzoïque correspondant (II)

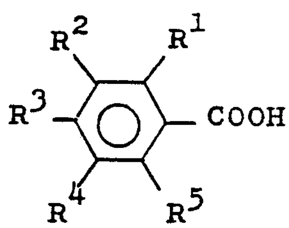

(II),

avec de l'alcool propargylique ou bien que l'on fait réagir de l'halogénure d'acide III

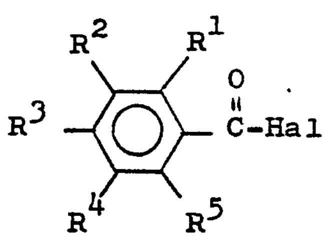

(III),

avec de l'alcool propargylique, en présence d'un accepteur d'acide, éventuellement en présence d'un solvant ou diluant.

3. Pesticide, contenant un composé selon la revendication 1.

4. Pesticide, contenant un support solide ou liquide et au moins un ester benzoique de formule I.

5. Utilisation d'esters benzoïques de formule I selon la revendication 1 - 3, comme insecticide, acaricide, en particulier ovicide.

6. Procédé de lutte contre les parasites, caractérisé par le fait que l'on fait agir une quantité efficace d'ester benzoïque de formule I selon la revendication 1 - 3 sur les parasites ou leur biotope.